# EUROPEAN PATENT APPLICATION

(11) **EP 3 564 358 A1**
(43) Date of publication of application: **06.11.2019**
(21) Application number: 17885711.6
(22) Date of filing: 22.12.2017
(51) Int. Cl.: C12N 1/15, C12N 11/16, C12N 15/00, C12N 15/09, C12P 1/00, C12P 1/02, C12P 7/64, C12P 19/14, C12P 19/30, C12P 21/00

(54) **TECHNOLOGY FOR SELECTIVE PROTEIN SECRETION IN FUNGUS**

(30) Priority: 27.12.2016 JP 2016252330
(71) Applicant: Kagoshima University, Kagoshima-shi, Kagoshima 890-8580 (JP); Kakui, Co., Ltd., Kagoshima 890-0081 (JP)
(72) Inventor: TAMAKI, Hisanori, Kagoshima-shi Kagoshima 890-8580 (JP); FUTAGAMI, Taiki, Kagoshima-shi Kagoshima 890-8580 (JP); IWAMOTO, Masataka, Kagoshima-shi Kagoshima 890-0081 (JP)
(74) Representative: J A Kemp LLP
(86) International application number: PCT/JP2017/046107
(87) International publication number: WO 2018/123856

(57) **Abstract**

It is an object of the present invention to provide a convenient method for obtaining a culture supernatant having a reduced amount of an undesired enzyme, a microorganism which may be used for the aforementioned method, a method for producing a target substance using the microorganism, and the like.

The present invention relates to a modified fungus expressing a fusion polynucleotide comprising a polynucleotide encoding a GPI-addition signal sequence linked to a polynucleotide encoding an endogenous secreted enzyme, a method for producing a target substance using the modified fungus, and the like.

SEQUENCE LISTING

<110>  Kagoshima University
       Kakui, Co., Ltd.

<120>  A technique for selective secretion of proteins in a fungi

<130>  PH-7217-PCT

<150>  JP 2016-252330
<151>  2016-12-27

<160>  20

<170>  PatentIn version 3.5

<210>  1
<211>  20
<212>  DNA
<213>  Artificial

<220>
<223>  Primer

<400>  1
atgcgttacc gaacagcagc                                                   20


<210>  2
<211>  20
<212>  DNA
<213>  Artificial

<220>
<223>  Primer

<400>  2
gccactgggc cctttgctag                                                   20


<210>  3
<211>  40
<212>  DNA
<213>  Artificial

<220>
<223>  Primer

<400>  3
ttgaggctgg gaagctcgac cgctaccgac agagtgctcg                             40


<210>  4
<211>  40
<212>  DNA
<213>  Artificial

<220>
<223>  Primer

<400>  4
cgagcactct gtcggtagcg gtcgagcttc ccagcctcaa                             40

<210>  5
<211>  40
<212>  DNA
<213>  Artificial

<220>
<223>  Primer

<400>  5
cttccaagcg gagcaggctc ttaccacagc tgggccatgc                              40


<210>  6
<211>  40
<212>  DNA
<213>  Artificial

<220>
<223>  Primer

<400>  6
gcatggccca gctgtggtaa gagcctgctc cgcttggaag                              40


<210>  7
<211>  39
<212>  DNA
<213>  Artificial

<220>
<223>  Primer

<400>  7
cttcaccctc ctcgcgctag ccaagagcgg attcctcag                               39


<210>  8
<211>  39
<212>  DNA
<213>  Artificial

<220>
<223>  Primer

<400>  8
ctgaggaatc cgctcttggc tagcgcgagg agggtgaag                               39


<210>  9
<211>  20
<212>  DNA
<213>  Artificial

<220>
<223>  Primer

<400>  9
ttatggcgcg cgaggagatc                                                    20


<210>  10
<211>  20

<212>  DNA
<213>  Artificial

<220>
<223>  Primer

<400>  10
cgctacgcgc tcagattcca                                                   20


<210>  11
<211>  843
<212>  PRT
<213>  Artificial

<220>
<223>  Synthetic construct

<400>  11

Met Arg Tyr Arg Thr Ala Ala Ala Leu Ala Leu Ala Thr Gly Pro Phe
1               5                   10                  15

Ala Arg Ala Asp Ser His Ser Thr Ser Gly Ala Ser Ala Glu Ala Val
            20                  25                  30

Val Pro Pro Ala Gly Thr Pro Trp Gly Thr Ala Tyr Asp Lys Ala Lys
        35                  40                  45

Ala Ala Leu Ala Lys Leu Asn Leu Gln Asp Lys Val Gly Ile Val Ser
    50                  55                  60

Gly Val Gly Trp Asn Gly Gly Pro Cys Val Gly Asn Thr Ser Pro Ala
65                  70                  75                  80

Ser Lys Ile Ser Tyr Pro Ser Leu Cys Leu Gln Asp Gly Pro Leu Gly
                85                  90                  95

Val Arg Tyr Ser Thr Gly Ser Thr Ala Phe Thr Pro Gly Val Gln Ala
            100                 105                 110

Ala Ser Thr Trp Asp Val Asn Leu Ile Arg Glu Arg Gly Gln Phe Ile
        115                 120                 125

Gly Glu Glu Val Lys Ala Ser Gly Ile His Val Ile Leu Gly Pro Val
    130                 135                 140

Ala Gly Pro Leu Gly Lys Thr Pro Gln Gly Gly Arg Asn Trp Glu Gly
145                 150                 155                 160

Phe Gly Val Asp Pro Tyr Leu Thr Gly Ile Ala Met Gly Gln Thr Ile
                165                 170                 175

Asn Gly Ile Gln Ser Val Gly Val Gln Ala Thr Ala Lys His Tyr Ile
            180                 185                 190

Leu Asn Glu Gln Glu Leu Asn Arg Glu Thr Ile Ser Ser Asn Pro Asp
        195                 200                 205

Asp Arg Thr Leu His Glu Leu Tyr Thr Trp Pro Phe Ala Asp Ala Val
    210                 215                 220

Gln Ala Asn Val Ala Ser Val Met Cys Ser Tyr Asn Lys Val Asn Thr
225                 230                 235                 240

Thr Trp Ala Cys Glu Asp Gln Tyr Thr Leu Gln Thr Val Leu Lys Asp
                245                 250                 255

Gln Leu Gly Phe Pro Gly Tyr Val Met Thr Asp Trp Asn Ala Gln His
            260                 265                 270

Thr Thr Val Gln Ser Ala Asn Ser Gly Leu Asp Met Ser Met Pro Gly
        275                 280                 285

Thr Asp Phe Asn Gly Asn Asn Arg Leu Trp Gly Pro Ala Leu Thr Asn
    290                 295                 300

Ala Val Asn Ser Asn Gln Val Pro Thr Ser Arg Val Asp Asp Met Val
305                 310                 315                 320

Thr Arg Ile Leu Ala Ala Trp Tyr Leu Thr Gly Gln Asp Gln Ala Gly
                325                 330                 335

Tyr Pro Ser Phe Asn Ile Ser Arg Asn Val Gln Gly Asn His Lys Thr
            340                 345                 350

Asn Val Arg Ala Ile Ala Arg Asp Gly Ile Val Leu Leu Lys Asn Asp
        355                 360                 365

Ala Asn Ile Leu Pro Leu Lys Lys Pro Ala Ser Ile Ala Val Val Gly
    370                 375                 380

Ser Ala Ala Ile Ile Gly Asn His Ala Arg Asn Ser Pro Ser Cys Asn
385                 390                 395                 400

Asp Lys Gly Cys Asp Asp Gly Ala Leu Gly Met Gly Trp Gly Ser Gly
                405                 410                 415

Ala Val Asn Tyr Pro Tyr Phe Val Ala Pro Tyr Asp Ala Ile Asn Thr
            420                 425                 430

Arg Ala Ser Ser Gln Gly Thr Gln Val Thr Leu Ser Asn Thr Asp Asn
        435                 440                 445

Thr Ser Ser Gly Ala Ser Ala Ala Arg Gly Lys Asp Val Ala Ile Val
    450                 455                 460

Phe Ile Thr Ala Asp Ser Gly Glu Gly Tyr Ile Thr Val Glu Gly Asn
465                 470                 475                 480

Ala Gly Asp Arg Asn Asn Leu Asp Pro Trp His Asn Gly Asn Ala Leu
                485                 490                 495

Val Gln Ala Val Ala Gly Ala Asn Ser Asn Val Ile Val Val Val His
            500                 505                 510

Ser Val Gly Ala Ile Ile Leu Glu Gln Ile Leu Ala Leu Pro Gln Val
        515                 520                 525

Lys Ala Val Val Trp Ala Gly Leu Pro Ser Gln Glu Ser Gly Asn Ala
    530                 535                 540

Leu Val Asp Val Leu Trp Gly Asp Val Ser Pro Ser Gly Lys Leu Val
545                 550                 555                 560

Tyr Thr Ile Ala Lys Ser Pro Asn Asp Tyr Asn Thr Arg Ile Val Ser
                565                 570                 575

Gly Gly Ser Asp Ser Phe Ser Glu Gly Leu Phe Ile Asp Tyr Lys His
            580                 585                 590

Phe Asp Asp Ala Asn Ile Thr Pro Arg Tyr Glu Phe Gly Tyr Gly Leu
        595                 600                 605

Ser Tyr Thr Lys Phe Asn Tyr Ser Arg Leu Ser Val Leu Ser Thr Ala
    610                 615                 620

Lys Ser Gly Pro Ala Thr Gly Ala Val Val Pro Gly Gly Pro Ser Asp
625                 630                 635                 640

Leu Phe Gln Asn Val Ala Thr Val Thr Val Asp Ile Ala Asn Ser Gly
                645                 650                 655

Gln Val Thr Gly Ala Glu Val Ala Gln Leu Tyr Ile Thr Tyr Pro Ser
            660                 665                 670

Ser Ala Pro Arg Thr Pro Pro Lys Gln Leu Arg Gly Phe Ala Lys Leu

675                 680                 685

Asn Leu Thr Pro Gly Gln Ser Gly Thr Ala Thr Phe Asn Ile Arg Arg
    690                 695                 700

Arg Asp Leu Ser Tyr Trp Asp Thr Ala Ser Gln Lys Trp Val Val Pro
705                 710                 715                 720

Ser Gly Ser Phe Gly Ile Ser Val Gly Ala Ser Ser Arg Asp Ile Arg
                725                 730                 735

Leu Thr Ser Thr Leu Ser Val Ala Val Glu Leu Pro Ser Leu Lys Asp
            740                 745                 750

Val Lys Gly Gly Val Thr Val Tyr Ser Thr Thr Asp Ile Ser Ala Phe
        755                 760                 765

Cys Gly Phe Phe Asn Asn Ala Lys Ser Lys Lys Ile Ile Gln Gly Gln
    770                 775                 780

Glu Ser Cys Lys Ser Asn Val Lys Ala Ala Asn Glu Gly Gly Ser Asp
785                 790                 795                 800

Gly Asp Ser Ser Thr Ser Ser Gly Lys Asn Gly Ser Ser Gly Asn Asp
                805                 810                 815

Thr Asp Asn Ala Val Ala Ala Leu Ser Val Asn Asn Met Leu Leu Gly
            820                 825                 830

Ala Ser Val Leu Ala Gly Met Ala Gln Leu Trp
        835                 840

<210>  12
<211>  2529
<212>  DNA
<213>  Artificial

<220>
<223>  Synthetic construct

<400>  12
atgcgttacc gaacagcagc tgcgctggca cttgccactg ggccctttgc tagggcagac      60

agtcactcaa catcgggggc ctcggctgag gcagttgtac ctcctgcagg gactccatgg     120

ggaaccgcgt acgacaaggc gaaggccgca ttggcaaagc tcaatctcca agataaggtc     180

ggcatcgtga gcggtgtcgg ctggaacggc ggtccttgcg ttggaaacac atctccggcc     240

tccaagatca gctatccatc gctatgcctt caagacggac ccctcggtgt tcgatactcg     300

acaggcagca cagcctttac gccgggcgtt caagcggcct cgacgtggga tgtcaatttg     360

atccgcgaac gtggacagtt catcggtgag gaggtgaagg cctcggggat tcatgtcata 420

cttggtcctg tggctgggcc gctgggaaag actccgcagg gcggtcgcaa ctgggagggc 480

ttcggtgtcg atccatatct cacgggcatt gccatgggtc aaaccatcaa cggcatccag 540

tcggtaggcg tgcaggcgac agcgaagcac tatatcctca acgagcagga gctcaatcga 600

gaaaccattt cgagcaaccc agatgaccga actctccatg agctgtatac ttggccattt 660

gccgacgcgg ttcaggccaa tgtcgcttct gtcatgtgct cgtacaacaa ggtcaatacc 720

acctgggcct gcgaggatca gtacacgctg cagactgtgc tgaaagacca gctggggttc 780

ccaggctatg tcatgacgga ctggaacgca cagcacacga ctgtccaaag cgcgaattct 840

gggcttgaca tgtcaatgcc tggcacagac ttcaacggta acaatcggct ctggggtcca 900

gctctcacca atgcggtaaa tagcaatcag gtccccacga gcagagtcga cgatatggtg 960

actcgtatcc tcgccgcatg gtacttgaca ggccaggacc aggcaggcta tccgtcgttc 1020

aacatcagca gaaatgttca aggaaaccac aagaccaatg tcagggcaat tgccagggac 1080

ggcatcgttc tgctcaagaa tgacgccaac atcctgccgc tcaagaagcc cgctagcatt 1140

gccgtcgttg gatctgccgc aatcattggt aaccacgcca gaaactcgcc ctcgtgcaac 1200

gacaaaggct gcgacgacgg ggccttgggc atgggttggg gttccggcgc cgtcaactat 1260

ccgtacttcg tcgcgcccta cgatgccatc aataccagag cgtcttcgca gggcacccag 1320

gttaccttga gcaacaccga caacacgtcc tcaggcgcat ctgcagcaag aggaaaggac 1380

gtcgccatcg tcttcatcac cgccgactcg ggtgaaggct acatcaccgt ggagggcaac 1440

gcgggcgatc gcaacaacct ggatccgtgg cacaacggca atgccctggt ccaggcggtg 1500

gccggtgcca acagcaacgt cattgttgtt gtccactccg ttggcgccat cattctggag 1560

cagattcttg ctcttccgca ggtcaaggcc gttgtctggg cgggtcttcc ttctcaggag 1620

agcggcaatg cgctcgtcga cgtgctgtgg ggagatgtca gcccttctgg caagctggtg 1680

tacaccattg cgaagagccc caatgactat aacactcgca tcgtttccgg cggcagtgac 1740

agcttcagcg agggactgtt catcgactat aagcacttcg acgacgccaa tatcacgccg 1800

cggtacgagt tcggctatgg actgtcttac accaagttca actactcacg cctctccgtc 1860

ttgtcgaccg ccaagtctgg tcctgcgact ggggccgttg tgccgggagg cccgagtgat 1920

ctgttccaga atgtcgcgac agtcaccgtt gacatcgcaa actctggcca agtgactggt 1980

gccgaggtag cccagctgta catcacctac ccatcttcag cacccaggac ccctccgaag 2040

cagctgcgag gctttgccaa gctgaacctc acgcctggtc agagcggaac agcaacgttc 2100

aacatccgac gacgagatct cagctactgg gacacggctt cgcagaaatg ggtggtgccg 2160

tcggggtcgt ttggcatcag cgtgggagcg agcagccggg atatcaggct gacgagcact 2220

ctgtcggtag cggtcgagct tcccagcctc aaggacgtca agggtggtgt taccgtctac 2280

tctaccaccg acatttctgc tttctgcggc ttcttcaaca atgctaagtc taagaagatt 2340

atccagggtc aggagtcctg caagagcaac gttaaggccg ccaatgaggg aggtagcgac 2400

ggtgactcca gcacttctag cggaaagaac ggcagcagcg gtaacgacac tgacaacgcc 2460

gtcgccgccc tcagcgtcaa caacatgctc ctcggcgcgt ccgttctcgc tggcatggcc 2520

cagctgtgg 2529

<210> 13
<211> 744
<212> PRT
<213> Tricoderma reesei

<400> 13

Met Arg Tyr Arg Thr Ala Ala Ala Leu Ala Leu Ala Thr Gly Pro Phe
1               5                   10                  15

Ala Arg Ala Asp Ser His Ser Thr Ser Gly Ala Ser Ala Glu Ala Val
            20                  25                  30

Val Pro Pro Ala Gly Thr Pro Trp Gly Thr Ala Tyr Asp Lys Ala Lys
        35                  40                  45

Ala Ala Leu Ala Lys Leu Asn Leu Gln Asp Lys Val Gly Ile Val Ser
    50                  55                  60

Gly Val Gly Trp Asn Gly Gly Pro Cys Val Gly Asn Thr Ser Pro Ala
65                  70                  75                  80

Ser Lys Ile Ser Tyr Pro Ser Leu Cys Leu Gln Asp Gly Pro Leu Gly
                85                  90                  95

Val Arg Tyr Ser Thr Gly Ser Thr Ala Phe Thr Pro Gly Val Gln Ala
            100                 105                 110

Ala Ser Thr Trp Asp Val Asn Leu Ile Arg Glu Arg Gly Gln Phe Ile
        115                 120                 125

Gly Glu Glu Val Lys Ala Ser Gly Ile His Val Ile Leu Gly Pro Val
    130                 135                 140

Ala Gly Pro Leu Gly Lys Thr Pro Gln Gly Gly Arg Asn Trp Glu Gly
145                 150                 155                 160

Phe Gly Val Asp Pro Tyr Leu Thr Gly Ile Ala Met Gly Gln Thr Ile
                165                 170                 175

Asn Gly Ile Gln Ser Val Gly Val Gln Ala Thr Ala Lys His Tyr Ile
            180                 185                 190

Leu Asn Glu Gln Glu Leu Asn Arg Glu Thr Ile Ser Ser Asn Pro Asp
        195                 200                 205

Asp Arg Thr Leu His Glu Leu Tyr Thr Trp Pro Phe Ala Asp Ala Val
    210                 215                 220

Gln Ala Asn Val Ala Ser Val Met Cys Ser Tyr Asn Lys Val Asn Thr
225                 230                 235                 240

Thr Trp Ala Cys Glu Asp Gln Tyr Thr Leu Gln Thr Val Leu Lys Asp
                245                 250                 255

Gln Leu Gly Phe Pro Gly Tyr Val Met Thr Asp Trp Asn Ala Gln His
            260                 265                 270

Thr Thr Val Gln Ser Ala Asn Ser Gly Leu Asp Met Ser Met Pro Gly
        275                 280                 285

Thr Asp Phe Asn Gly Asn Asn Arg Leu Trp Gly Pro Ala Leu Thr Asn
    290                 295                 300

Ala Val Asn Ser Asn Gln Val Pro Thr Ser Arg Val Asp Asp Met Val
305                 310                 315                 320

Thr Arg Ile Leu Ala Ala Trp Tyr Leu Thr Gly Gln Asp Gln Ala Gly
                325                 330                 335

Tyr Pro Ser Phe Asn Ile Ser Arg Asn Val Gln Gly Asn His Lys Thr
            340                 345                 350

Asn Val Arg Ala Ile Ala Arg Asp Gly Ile Val Leu Leu Lys Asn Asp
        355                 360                 365

Ala Asn Ile Leu Pro Leu Lys Lys Pro Ala Ser Ile Ala Val Val Gly
    370                 375                 380

Ser Ala Ala Ile Ile Gly Asn His Ala Arg Asn Ser Pro Ser Cys Asn
385                 390                 395                 400

Asp Lys Gly Cys Asp Asp Gly Ala Leu Gly Met Gly Trp Gly Ser Gly
                405                 410                 415

Ala Val Asn Tyr Pro Tyr Phe Val Ala Pro Tyr Asp Ala Ile Asn Thr
            420                 425                 430

Arg Ala Ser Ser Gln Gly Thr Gln Val Thr Leu Ser Asn Thr Asp Asn
        435                 440                 445

Thr Ser Ser Gly Ala Ser Ala Ala Arg Gly Lys Asp Val Ala Ile Val
    450                 455                 460

Phe Ile Thr Ala Asp Ser Gly Glu Gly Tyr Ile Thr Val Glu Gly Asn
465                 470                 475                 480

Ala Gly Asp Arg Asn Asn Leu Asp Pro Trp His Asn Gly Asn Ala Leu
                485                 490                 495

Val Gln Ala Val Ala Gly Ala Asn Ser Asn Val Ile Val Val Val His
            500                 505                 510

Ser Val Gly Ala Ile Ile Leu Glu Gln Ile Leu Ala Leu Pro Gln Val
        515                 520                 525

Lys Ala Val Val Trp Ala Gly Leu Pro Ser Gln Glu Ser Gly Asn Ala
    530                 535                 540

Leu Val Asp Val Leu Trp Gly Asp Val Ser Pro Ser Gly Lys Leu Val
545                 550                 555                 560

Tyr Thr Ile Ala Lys Ser Pro Asn Asp Tyr Asn Thr Arg Ile Val Ser
                565                 570                 575

Gly Gly Ser Asp Ser Phe Ser Glu Gly Leu Phe Ile Asp Tyr Lys His
            580                 585                 590

Phe Asp Asp Ala Asn Ile Thr Pro Arg Tyr Glu Phe Gly Tyr Gly Leu
        595                 600                 605

Ser Tyr Thr Lys Phe Asn Tyr Ser Arg Leu Ser Val Leu Ser Thr Ala
    610                 615                 620

Lys Ser Gly Pro Ala Thr Gly Ala Val Val Pro Gly Gly Pro Ser Asp
625                 630                 635                 640

Leu Phe Gln Asn Val Ala Thr Val Thr Val Asp Ile Ala Asn Ser Gly
                645                 650                 655

Gln Val Thr Gly Ala Glu Val Ala Gln Leu Tyr Ile Thr Tyr Pro Ser
            660                 665                 670

Ser Ala Pro Arg Thr Pro Pro Lys Gln Leu Arg Gly Phe Ala Lys Leu

675                 680                 685

Asn Leu Thr Pro Gly Gln Ser Gly Thr Ala Thr Phe Asn Ile Arg Arg
    690                 695                 700

Arg Asp Leu Ser Tyr Trp Asp Thr Ala Ser Gln Lys Trp Val Val Pro
705                 710                 715                 720

Ser Gly Ser Phe Gly Ile Ser Val Gly Ala Ser Ser Arg Asp Ile Arg
                725                 730                 735

Leu Thr Ser Thr Leu Ser Val Ala
            740

<210>  14
<211>  2232
<212>  DNA
<213>  Tricoderma reesei

<400>  14
atgcgttacc gaacagcagc tgcgctggca cttgccactg ggccctttgc tagggcagac      60

agtcactcaa catcgggggc ctcggctgag gcagttgtac ctcctgcagg gactccatgg     120

ggaaccgcgt acgacaaggc gaaggccgca ttggcaaagc tcaatctcca agataaggtc     180

ggcatcgtga gcggtgtcgg ctggaacggc ggtccttgcg ttggaaacac atctccggcc     240

tccaagatca gctatccatc gctatgcctt caagacggac ccctcggtgt tcgatactcg     300

acaggcagca cagcctttac gccgggcgtt caagcggcct cgacgtggga tgtcaatttg     360

atccgcgaac gtggacagtt catcggtgag gaggtgaagg cctcggggat tcatgtcata     420

cttggtcctg tggctgggcc gctgggaaag actccgcagg gcggtcgcaa ctgggagggc     480

ttcggtgtcg atccatatct cacgggcatt gccatgggtc aaaccatcaa cggcatccag     540

tcggtaggcg tgcaggcgac agcgaagcac tatatcctca acgagcagga gctcaatcga     600

gaaaccattt cgagcaaccc agatgaccga actctccatg agctgtatac ttggccattt     660

gccgacgcgg ttcaggccaa tgtcgcttct gtcatgtgct cgtacaacaa ggtcaatacc     720

acctgggcct gcgaggatca gtacacgctg cagactgtgc tgaaagacca gctggggttc     780

ccaggctatg tcatgacgga ctggaacgca cagcacacga ctgtccaaag cgcgaattct     840

gggcttgaca tgtcaatgcc tggcacagac ttcaacggta acaatcggct ctggggtcca     900

gctctcacca atgcggtaaa tagcaatcag gtccccacga gcagagtcga cgatatggtg     960

actcgtatcc tcgccgcatg gtacttgaca ggccaggacc aggcaggcta tccgtcgttc    1020

aacatcagca gaaatgttca aggaaaccac aagaccaatg tcagggcaat tgccagggac    1080

ggcatcgttc tgctcaagaa tgacgccaac atcctgccgc tcaagaagcc cgctagcatt    1140

gccgtcgttg gatctgccgc aatcattggt aaccacgcca gaaactcgcc ctcgtgcaac 1200

gacaaaggct gcgacgacgg ggccttgggc atgggttggg gttccggcgc cgtcaactat 1260

ccgtacttcg tcgcgcccta cgatgccatc aataccagag cgtcttcgca gggcacccag 1320

gttaccttga gcaacaccga caacacgtcc tcaggcgcat ctgcagcaag aggaaaggac 1380

gtcgccatcg tcttcatcac cgccgactcg ggtgaaggct acatcaccgt ggagggcaac 1440

gcgggcgatc gcaacaacct ggatccgtgg cacaacggca atgccctggt ccaggcggtg 1500

gccggtgcca acagcaacgt cattgttgtt gtccactccg ttggcgccat cattctggag 1560

cagattcttg ctcttccgca ggtcaaggcc gttgtctggg cgggtcttcc ttctcaggag 1620

agcggcaatg cgctcgtcga cgtgctgtgg ggagatgtca gcccttctgg caagctggtg 1680

tacaccattg cgaagagccc caatgactat aacactcgca tcgtttccgg cggcagtgac 1740

agcttcagcg agggactgtt catcgactat aagcacttcg acgacgccaa tatcacgccg 1800

cggtacgagt tcggctatgg actgtcttac accaagttca actactcacg cctctccgtc 1860

ttgtcgaccg ccaagtctgg tcctgcgact ggggccgttg tgccgggagg cccgagtgat 1920

ctgttccaga atgtcgcgac agtcaccgtt gacatcgcaa actctggcca agtgactggt 1980

gccgaggtag cccagctgta catcacctac ccatcttcag cacccaggac ccctccgaag 2040

cagctgcgag gctttgccaa gctgaacctc acgcctggtc agagcggaac agcaacgttc 2100

aacatccgac gacgagatct cagctactgg gacacggctt cgcagaaatg ggtggtgccg 2160

tcggggtcgt ttggcatcag cgtgggagcg agcagccggg atatcaggct gacgagcact 2220

ctgtcggtag cg 2232

<210> 15
<211> 99
<212> PRT
<213> Tricoderma reesei

<400> 15

Val Glu Leu Pro Ser Leu Lys Asp Val Lys Gly Gly Val Thr Val Tyr
1               5                   10                  15

Ser Thr Thr Asp Ile Ser Ala Phe Cys Gly Phe Phe Asn Asn Ala Lys
            20                  25                  30

Ser Lys Lys Ile Ile Gln Gly Gln Glu Ser Cys Lys Ser Asn Val Lys
        35                  40                  45

Ala Ala Asn Glu Gly Gly Ser Asp Gly Asp Ser Ser Thr Ser Ser Gly
    50                  55                  60

Lys Asn Gly Ser Ser Gly Asn Asp Thr Asp Asn Ala Val Ala Ala Leu

65                  70                  75                  80

Ser Val Asn Asn Met Leu Leu Gly Ala Ser Val Leu Ala Gly Met Ala
                85                  90                  95

Gln Leu Trp

<210>  16
<211>  297
<212>  DNA
<213>  Tricoderma reesei

<400>  16
gtcgagcttc ccagcctcaa ggacgtcaag ggtggtgtta ccgtctactc taccaccgac      60

atttctgctt tctgcggctt cttcaacaat gctaagtcta agaagattat ccagggtcag     120

gagtcctgca agagcaacgt taaggccgcc aatgagggag gtagcgacgg tgactccagc     180

acttctagcg gaaagaacgg cagcagcggt aacgacactg acaacgccgt cgccgccctc     240

agcgtcaaca acatgctcct cggcgcgtcc gttctcgctg gcatggccca gctgtgg        297

<210>  17
<211>  100
<212>  PRT
<213>  Aspergillus kawachii

<400>  17

Val Ser Leu Ser Ser Leu Thr Glu Val Lys Gly Gly Phe Asn Met Gln
1               5                   10                  15

Ser Thr Gly Asn Phe Ser Cys Ser Ala Phe Lys Thr Leu Arg Glu Asn
            20                  25                  30

Asn Val Ile Arg Gly Thr Tyr Thr Cys Lys Ala Ser Thr Ser Asp Pro
        35                  40                  45

Thr Thr Ser Asp Gly Ser Ser Gly Thr Ser Ser Ser Thr Ser Ser Ser
    50                  55                  60

Ser Ser Ser Ser Ser Thr Ser Ser Asp Ser Ala Ser Val Met Asn Val
65                  70                  75                  80

Ala Asn Met Pro Ala Leu Gly Leu Ala Ala Val Leu Gly Gly Leu Ile
                85                  90                  95

Gln Tyr Ala Leu
            100

<210>  18
<211>  300
<212>  DNA
<213>  Aspergillus kawachii

<400>  18
gtttccctct cttccctgac cgaggttaag ggtggcttca acatgcagag cactggtaac        60

ttcagctgca gcgccttcaa gaccctgcgt gagaacaacg tcatccgtgg tacctacacc       120

tgcaaggcca gcacctccga ccccaccacc agcgatggat cctctggcac ctcttcctcc       180

accagcagca gcagcagctc ttcctccacc agcagcgaca gcgcctccgt catgaacgtg       240

gccaacatgc ccgctcttgg tcttgctgcc gtcctcggtg gtctgatcca gtacgctctg       300


<210>  19
<211>  100
<212>  PRT
<213>  Penicillium chrysogenum

<400>  19

Gly Thr Phe Asn Lys Leu Ser Leu Gly Ala Leu Lys Asp Val Glu Gly
1               5                   10                  15


Ser Phe Asn Met Gln Ser Thr Arg Gly Asn Phe Ser Cys Asp Asp Leu
            20                  25                  30


Arg Lys Leu Lys Asn Asp Asp Val Ile Lys Gly Ser Phe Lys Cys Asp
        35                  40                  45


Ala Thr Asn Ala Asn Pro Thr Thr Ser Lys Gly Gly Ser Gly Thr Ser
    50                  55                  60


Ser Ser Asp Ser Ser Ser Glu Thr Ser Glu Gly Ala Ala Phe Met Thr
65                  70                  75                  80


Gly Ala Asn Val Pro Val Val Gly Leu Ala Ala Leu Phe Gly Ala Leu
                85                  90                  95


Ala Gln Leu Leu
            100


<210>  20
<211>  300
<212>  DNA
<213>  Penicillium chrysogenum

<400>  20
ggtaccttca acaagctttc tcttggtgct ctcaaggatg tcgagggtag cttcaacatg        60

cagagcactc gcggcaactt cagctgtgac gatctccgca agctgaagaa cgacgatgtc       120

atcaagggta gcttcaagtg tgatgccacc aacgccaacc ccaccacttc caagggcgga       180

tctggcacct ccagctctga ctcctcttcc gagaccagcg aaggtgctgc cttcatgacc 240

ggcgccaacg tccccgtcgt cggcctcgcc gctctcttcg gtgcccttgc ccagcttctg 300

## Description

### Technical Field

The present invention relates to a modified fungus expressing a fusion polynucleotide comprising a polynucleotide encoding a GPI-addition signal sequence linked to a polynucleotide encoding an endogenous secreted enzyme, a method for producing a target substance using the modified fungus, and the like.

### Background Art

Since fungi, in particular, filamentous fungi are capable of secreting and producing a variety of enzymes, the culture supernatant thereof has been used as an enzyme preparation. However, when the culture supernatant of a fungus is used as an enzyme preparation, the contamination with an undesired enzyme derived from the fungus, for example, an enzyme having an activity of degrading a target substance is problematic. In order to remove such an undesired enzyme from the culture supernatant, a purification or separation process, etc. is necessary. Thus, a fungus-derived enzyme preparation has had two serious problems, namely, expensiveness and a difficulty in stable supply due to unstable production amount. Moreover, there has been a method of using a strain, in which a gene encoding the undesired enzyme is disrupted, as a method of reducing the amount of an undesired enzyme. In this method, however, the proliferative ability of the fungus may be significantly reduced. Accordingly, it has been desired to develop a method for obtaining a culture supernatant, having a reduced amount of an undesired enzyme without significantly impairing the proliferative ability of a fungus.

On the other hand, GPI (glycosylphosphatidylinositol) is a glycolipid added to the C-terminus of a protein by post-translational modification, and GPI has the function of immobilizing (anchoring) a certain type of protein on the surface of a cell. To date, it has been demonstrated that GPI is used to display a protein on the surface of a cell in yeast (Patent Literature 1). However, application of the GPI to an endogenous secreted enzyme has not been envisaged at all so far.

### Citation List

### Patent Literature

Patent Literature 1: JP Patent Publication (Kohyo) No. 2012-511920 A

### Summary of Invention

### Technical Problem

It is an object of the present invention to provide: a method for obtaining a culture supernatant having a reduced amount of an undesired enzyme without significantly impairing the proliferative ability of a fungus; a microorganism which may be used in the aforementioned method; a method for producing a target substance using the microorganism or the culture supernatant; and the like.

### Solution to Problem

The present inventors have found that, an endogenous secreted enzyme remains on the surface layer of a cell by adding a GPI-addition signal sequence to a gene encoding the endogenous secreted enzyme, so that the amount of the endogenous secreted enzyme in a culture supernatant is reduced, thereby completing the present invention.

Therefore, the invention of the present application includes the following aspects.
(1) A modified fungus, expressing a fusion polynucleotide comprising an exogenous polynucleotide encoding a glycosylphosphatidylinositol (GPI)-addition signal sequence linked to the 3'-terminal side of a polynucleotide encoding an endogenous secreted enzyme, and thereby, the secreted enzyme is anchored on the cell surface by GPI.
(2) The fungus according to the above (1), which belongs to filamentous fungi.
(3) The fungus according to the above (2), which belongs to filamentous fungi selected from the group consisting of the genus Trichoderma, the genus Aspergillus, the genus Penicillium, the genus Monascus, and the genus Thermoascus.
(4) The fungus according to the above (3), which belongs to the species *Trichoderma reesei.*
(5) The fungus according to any one of the above (1) to (4), wherein the secreted enzyme is selected from the group consisting of glycosidase, lipase, protease, and nuclease.
(6) The fungus according to any one of the above (1) to (5), which is used in the production of a target substance, wherein the target substance and the secreted enzyme are selected from the group consisting of the following combinations:
   (a) the target substance is a sugar and the secreted enzyme is glycosidase;
   (b) the target substance is a lipid and the secreted enzyme is lipase;
   (c) the target substance is a protein and the secreted enzyme is protease; and
   (d) the target substance is a nucleic acid and the secreted enzyme is nuclease.
(7) The fungus according to the above (6), wherein the target substance is cellobiose and the secreted enzyme is β glucosidase.
(8) A method for producing a fungal culture supernatant having a reduced amount of an endogenous secreted enzyme, comprising:
   a step of culturing the fungus according to any one of the above (1) to (5) in a medium; and
   a step of collecting a culture supernatant having a reduced amount of an endogenous secreted enzyme from the cultured fungus.
(9) A method for producing a target substance, comprising
   a step of contacting a raw material substance with the fungus according to any one of the above (1) to (5) or the fungal culture supernatant according to the above (8), to produce a target substance from the raw material substance.
(10) The method according to the above (9),
   wherein the target substance and a secreted enzyme are any of the following combinations:
   (a) the target substance is a sugar and the secreted enzyme is glycosidase;
   (b) the target substance is a lipid and the secreted enzyme is lipase;
   (c) the target substance is a protein and the secreted enzyme is protease; and
   (d) the target substance is a nucleic acid and the secreted enzyme is nuclease.
(11) The method according to the above (10), wherein the target substance is cellobiose and the secreted enzyme is β glucosidase.
(12) The method according to any one of the above (9) to (11), comprising a step of collecting the target substance.
(13) A method for producing a modified fungus, in which an endogenous secreted enzyme is anchored on the surface of a cell by glycosylphosphatidylinositol (GPI),
   wherein the method comprises a step of modifying a fungus to express a fusion polynucleotide comprising an exogenous polynucleotide encoding a GPI-addition signal sequence linked to the 3'-terminal side of a polynucleotide encoding the endogenous secreted enzyme.
(14) The method according to the above (13), wherein the fungus belongs to the species *Trichoderma reesei.*
(15) The method according to the above (13) or (14), wherein the secreted enzyme is selected from the group consisting of glycosidase, lipase, protease, and nuclease.

The present description includes the disclosure of Japanese Patent Application No. 2016-252330, to which the present application claims priority.

In one embodiment of the present invention, a culture supernatant having a reduced amount of an undesired endogenous secreted enzyme can be obtained, and this culture supernatant may be directly used as an enzyme preparation. In addition, in one embodiment, , a combination of secreted enzymes can be easily customized by applying the present method to various enzymes secreted by fungi, and thus, the present method can be applied to the production of an enzyme preparation and the production of a substance, which are suitable for various purposes.

### Brief Description of Drawings

[Figure 1] Figure 1 shows the results of electrophoresis, after PCR performed on the wild-type strain and the mutant strain (Bgll-GPI strain) prepared in the Examples. It was confirmed that an exogenous polynucleotide encoding a GPI-addition signal sequence was added to the bgl1 gene in the Bgll-GPI strain, by observing the shift-up caused by sequence insertion in the Bgll-GPI strain.
[Figure 2] Figure 2 shows the weight of a freeze-dried cell mass (A) of each of the wild-type strain and the mutant strain (Bgll-GPI strain) prepared in the Examples, and the measurement results (B) of the Bgll activity of the culture supernatant of each strain.
[Figure 3] Figure 3 shows the measurement results of the CMCase activity (A), CBH activity (B), and Bgll activity (C) of the culture supernatant of each of the wild-type strain and the mutant strain (Bgll-GPI strain) prepared in the Examples.
[Figure 4] Figure 4 shows a change over time in the ratio of glucose/cellobiose produced from cellulose in the wild-type strain and the mutant strain (Bgll-GPI strain) prepared in the Examples.

### Description of Embodiments

### 1. Modified fungus

In one aspect, the present invention relates to a modified fungus.

The modified fungus of the present invention is genetically modified by linking an exogenous polynucleotide encoding a glycosylphosphatidylinositol (GPI)-addition signal sequence to the 3'-terminal side of a polynucleotide encoding an endogenous secreted enzyme.

As used herein, the "endogenous secreted enzyme" means all enzymes originally secreted by a genetically unmodified fungus, in particular, enzymes whose secretion is undesirable. For example, when the modified fungus of the present invention is used for producing a substance, examples of the enzymes whose secretion is "undesirable" include an enzyme having an activity of degrading the target substance, an enzyme inhibiting the production of the target substance, and an enzyme interfering the activity of the target substance. Specific examples of the endogenous secreted enzyme include, but are not limited to, glycosidase (in particular, glucosidase), lipase, protease, and nuclease, preferably, glycosidase (in particular, glucosidase), lipase, or protease; and more preferably, glycosidase (in particular, glucosidase), such as cellobiohydrolase (EC3.2.1.91), endoglucanase (EC3.2.1.4), and β glucosidase (EC 3.2.1.21). When the modified fungus is *Trichoderma reesei,* the endogenous secreted enzyme is preferably β glucosidase (EC 3.2.1.21), more preferably secretory β glucosidase that is at least one of Bgll, Cel3b, Cel3e, Cel3f, and Cel3g, and particularly preferably Bgll. The activity of these enzymes can be measured according to a method known to a person skilled in the art, for example, by using a substrate that is degraded by these enzymes to generate a fluorescent substance or a light-absorbing substance. For example, the activity of β glucosidase can be measured by using a substrate degraded by an enzyme having β glucosidase activity, such as p-nitrophenyl-β-D-glucopyranoside, to generate a light-absorbing substance.

The sequences of endogenous secreted enzymes are available from public database (e.g., NCBI (U.S.A.), DDBJ (Japan), and EMBL (Europe)). For instance, Bgll (β glucosidase 1) of *Trichoderma reesei* comprises the amino acid sequence as set forth in SEQ ID NO: 13. In addition, a polynucleotide encoding Bgll of *Trichoderma reesei* comprises the polynucleotide sequence as set forth in SEQ ID NO: 14. Bgll may comprise functional equivalents of the above-described amino acid sequence, such as, for example, (i) an amino acid sequence having an identity of, for example, 70% or more, 80% or more, preferably 90% or more, 95% or more, 97% or more, 98% or more, or 99% or more, to the amino acid sequence as set forth in SEQ ID NO: 13, (ii) an amino acid sequence comprising the amino acid sequence as set forth in SEQ ID NO: 13 in which one or multiple amino acids are added, deleted, and/or substituted, or (iii) an amino acid sequence encoded by a polynucleotide that hybridizes under stringent conditions to a complementary strand of a polynucleotide comprising a polynucleotide sequence encoding the amino acid sequence as set forth in SEQ ID NO: 13. Likewise, a polynucleotide encoding Bgll may comprise functional equivalents of the above-described polynucleotide sequence, such as, for example, (i) a polynucleotide sequence having an identity of, for example, 70% or more, 80% or more, preferably 90% or more, 95% or more, 97% or more, 98% or more, or 99% or more, to the polynucleotide sequence as set forth in SEQ ID NO: 14, (ii) a polynucleotide sequence comprising the polynucleotide sequence as set forth in SEQ ID NO: 14 in which one or multiple nucleotides are added, deleted, and/or substituted, or (iii) the polynucleotide sequence of a polynucleotide that hybridizes under stringent conditions to a complementary strand of a polynucleotide comprising the polynucleotide sequence as set forth in SEQ ID NO: 14.

As used herein, the identity value regarding amino acid sequences and nucleotide sequences indicates a value calculated using software for calculating identity among multiple sequences (e.g., FASTA, DANASYS, and BLAST), with default settings. For details of a method of determining an identity, please refer to, for example, Altschul et al, Nuc. Acids. Res. 25, 3389-3402, 1977 and Altschul et al, J. Mol. Biol. 215, 403-410, 1990.

In addition, as used herein, the range of "one or multiple" regarding an amino acid sequence and a nucleotide sequence indicates 1 to 10, preferably 1 to 7, more preferably 1 to 5, particularly preferably several, and for example, 1 to 4, 1 to 3, or 1 or 2.

Moreover, as used herein, the "stringent conditions" mean conditions under which a so-called specific hybrid is formed and a non-specific hybrid is not formed. The stringent conditions can be determined, as appropriate, with reference to, for example, Green and Sambrook, Molecular Cloning, 4th Ed (2012), Cold Spring Harbor Laboratory Press. Specifically, the stringent conditions may be defined based on the temperature and the concentration of salts in a solution applied during Sothern hybridization, and based on the temperature and the concentration of salts in a solution applied upon the washing step during Sothern hybridization. In more details, examples of the stringent conditions include a sodium concentration of 25 to 500mM, preferably 25 to 300mM, and a temperature of 42°C to 68°C, preferably 42°C to 65°C. More specifically, examples of the stringent conditions include a sodium concentration of 5 x SSC (wherein 1 x SSC consists of 150mM NaCl and 15mM sodium citrate) and a temperature of 42°C.

As used herein, glycosylphosphatidylinositol (hereinafter also referred to as simple "GPI") means a glycolipid comprising an oligosaccharide chain and an inositol phospholipid, which has the function of immobilizing (anchoring) a certain type of protein on the surface of a cell. GPI basically comprises ethanolamine, phosphoric acid, trimannoside, glucosamine, and inositol phospholipid in this order (see, for example, Hiroh IKEZAWA, Biol. Pharm. Bull. 25(4) 409-417). It has been known that the side chain of a sugar chain moiety and the molecular species of a lipid moiety of GPI vary depending on an organism species, a cell or a protein.

GPI is synthesized in the endoplasmic reticulum, and is linked to the C-terminus of a protein. In general, a GPI-anchored protein comprises a GPI-addition signal sequence (also referred to as a "GPI-addition signal") at the C-terminus, as well as a signal peptide at the N-terminus. This GPI-addition signal sequence is cleaved, and an ethanolamine moiety of GPI synthesized in the endoplasmic reticulum is then linked to the thus newly generated carboxy terminus (referred to as "ω site"). Thereafter, the GPI-anchored protein is transferred to the surface of a cell, while it undergoes further modifications.

In the modified fungus of the present invention, a secreted enzyme is anchored on the surface of a cell by GPI by linking an exogenous polynucleotide encoding a GPI-addition signal sequence to the 3'-terminal side of a polynucleotide encoding the endogenous secreted enzyme. The exogenous polynucleotide encoding the GPI-addition signal sequence may be directly linked to the 3'-terminal side of the polynucleotide encoding an endogenous secreted enzyme. Alternatively, they may be then linked to each other with adding a suitable linker sequence, for example, approximately 1 to 300, 5 to 200, or 10 to 100 nucleotides to the exogenous polynucleotide without shifting the reading frame.

As a result of the above-described modification, in the modified fungus of the present invention, the secretion amount of the endogenous secreted enzyme can be reduced in comparison to that of a wild-type fungus. In the modified fungus of the present invention, only one polynucleotide encoding an endogenous secreted enzyme may be modified, or multiple polynucleotides encoding endogenous secreted enzymes may be modified. When multiple polynucleotides encoding endogenous secreted enzymes are modified, for example, 2, 3, 4, 5 or more polynucleotides encoding endogenous secreted enzymes may be modified.

The degree of the secretion amount of the endogenous secreted enzyme that is reduced by the above-described modification is not particularly limited, but for example, the expression level may be 90% or less, 80% or less, 70% or less, 60% or less, preferably 50% or less, 40% or less, 30% or less, 20% or less, or 10% or less, compared to the expression level of a wild-type fungus. Moreover, the secretion of the endogenous secreted enzyme may be completely lost.

In the modified fungus of the present invention, the secretion amount of the endogenous secreted enzyme can be reduced. However, since the endogenous secreted enzyme is present on the surface of a cell and the expression itself is not reduced, it is considered that a reduction in the secretion amount of the endogenous secreted enzyme gives less influence on the growth of the fungus, compared with gene knockout or suppression of the expression.

The viability of the modified fungus of the present invention may be equivalent to that of a wild-type fungus, or may also be inferior to that of a wild-type fungus. For example, the viability of the modified fungus of the present invention may be 50% or more, 60% or more, 70% or more, 80% or more, preferably 90% or more, 95% or more, 98% or more, 99% or more, or 100% or more of that of a wild-type fungus, as measured by the weight (g) of a freeze-dried cell mass after the culture, the turbidity (OD₆₆₀) after cultivation or at specific time points during cultivation, or the viable cell count (cfu/ml) after cultivation or at specific time points during cultivation, preferably, as measured by the weight (g) of a freeze-dried cell mass after the culture.

As used herein, the type and sequence of the exogenous polynucleotide encoding a GPI-addition signal sequence are not particularly limited. As used herein, the "exogenous polynucleotide" means a polynucleotide that is not included in a natural polynucleotide encoding the "endogenous secreted enzyme." Accordingly, the "exogenous polynucleotide" may be derived from the same or different species as an organism expressing the "endogenous secreted enzyme". Preferably, the "exogenous polynucleotide" is derived from the same genus as an organism expressing the "endogenous secreted enzyme," and more preferably, is derived from the same species as an organism expressing the "endogenous secreted enzyme."

The GPI-addition signal sequence does not have a consensus sequence. A majority of GPI-addition signal sequences have the following four characteristics: (i) an amino acid having a small side chain is used as a site to which the ethanolamine moiety of GPI is linked (ω site); (ii) amino acids having a small side chain, such as Gly, Ala and Ser, are also used as an amino acid at ω+2 (two residues from the ω site on the C-terminal side, the same hereinafter); (iii) there is a hydrophilic region consisting of 6 to 10 amino acids from ω+3; and (iv) there is a hydrophobic region consisting of the subsequent 10 to 20 amino acids to the C-terminus (Morihisa FUJITA, Biochemistry, Vol. 85, No. 11, pp. 985-995, 2013). In recent years, a large number of programs predicting the GPI-addition signal sequence have been published. Further, a person skilled in the art can easily obtain such GPI-addition signal sequences from the public database.

Examples of the GPI-addition signal sequence include a GPI-addition signal sequence comprising the amino acid sequence as set forth in SEQ ID NO: 15 derived from *Trichoderma reesei,* a GPI-addition signal sequence comprising the amino acid sequence as set forth in SEQ ID NO: 17 derived from *Aspergillus kawachii,* and a GPI-addition signal sequence comprising the amino acid sequence as set forth in SEQ ID NO: 19 derived from *Penicillium chrysogenum.* Other examples of the GPI-addition signal sequence include (i) an amino acid sequence having an identity of, for example, 70% or more, 80% or more, preferably 90% or more, 95% or more, 97% or more, 98% or more, or 99% or more, to the amino acid sequence as set forth in SEQ ID NO: 15, 17, or 19, (ii) an amino acid sequence comprising the amino acid sequence as set forth in SEQ ID NO: 15, 17, or 19 in which one or multiple amino acids are added, deleted, and/or substituted, and (iii) an amino acid sequence encoded by a polynucleotide that hybridizes under stringent conditions to a complementary strand of a polynucleotide comprising a polynucleotide sequence encoding the amino acid sequence as set forth in SEQ ID NO: 15, 17, or 19.

Example of the polynucleotide encoding the GPI-addition signal sequence include polynucleotides comprising the nucleotide sequences as set forth in SEQ ID NOS: 16, 18, and 20 that encode the amino acid sequences as set forth in SEQ ID NOS: 15, 17, and 19, respectively. Other examples of the polynucleotide encoding the GPI-addition signal sequence include (i) a polynucleotide sequence having an identity of, for example, 70% or more, 80% or more, preferably 90% or more, 95% or more, 97% or more, 98% or more, or 99% or more, to the polynucleotide sequence as set forth in SEQ ID NO: 16, 18, or 20, (ii) a polynucleotide sequence comprising the polynucleotide sequence as set forth in SEQ ID NO: 16, 18, or 20 in which one or multiple nucleotides are added, deleted, and/or substituted, and (iii) the polynucleotide sequence of a polynucleotide that hybridizes under stringent conditions to a complementary strand of a polynucleotide comprising the polynucleotide sequence as set forth in SEQ ID NO: 16, 18, or 20.

The genus and species of a fungus from which the modified fungus of the present invention is derived are not particularly limited. It is preferably a yeast or a filamentous fungus. Examples of the yeast includes that selected from the group consisting of the genus Saccharomyces, the genus Kluyveromyces, the genus Candida, the genus Pichia, the genus Schizosaccharomyces, the genus Hansenula, and the genus Yarrowia, for example, *Saccharomyces cerevisiae, Candida albicans,* and *Pichia pastoris.*

The modified fungus is preferably a filamentous fungus. The filamentous fungus includes, but not limited to, that selected from the group consisting of the genus Trichoderma, the genus Aspergillus, the genus Penicillium, the genus Monascus, the genus Thermoascus, the genus Cephalosporium, the genus Acremonium, and the genus Neurospora, preferably, filamentous fungus of the genus selected from the group consisting of the genus Trichoderma, the genus Aspergillus, the genus Penicillium, the genus Monascus, and the genus Thermoascus, for example, *Trichoderma reesei, Aspergillus kawachii,* and *Penicillium chrysogenum.* The modified fungus of the present invention is particularly preferably a filamentous fungus of the genus Trichoderma, such as *Trichoderma reesei, Trichoderma harzianum, Trichoderma koningii, Trichoderma longibrachiatum, Trichoderma viride,* or *Trichoderma atroviride,* and is more preferably *Trichoderma reesei.* The fungal strain from which the modified fungus of the present invention is derived is not limited. It may be an isolated strain, or may also be a strain obtained from a depository such as ATCC or NBRC, for example, *Trichoderma reesei* NBRC 31327.

The modified fungus of the present invention can be prepared by any given method known to a person skilled in the art (for details of genetic recombination technology, see, for example, Sambrook et al., "Molecular Cloning, A Laboratory Manual fourth edition," Cold Spring Harber Laboratory Press, 2012). The modified fungus of the present invention can be prepared, for example, according to the following method for producing a modified fungus.

In one aspect, the present invention relates to a method for producing the above-described modified fungus, comprising the endogenous secreted enzyme is anchored on the surface of a cell by GPI. The method for producing a modified fungus comprises a step of modifying a fungus to expresses a fusion polynucleotide comprising an exogenous polynucleotide encoding a glycosylphosphatidylinositol (GPI)-addition signal sequence linked to the 3'-terminal side of a polynucleotide encoding an endogenous secreted enzyme.

For instance, the modification step can be carried out by introducing into fungal cells wild-type, a gene cassette comprising an exogenous polynucleotide encoding a glycosylphosphatidylinositol (GPI)-addition signal sequence flanked at 5'end and 3'end by, DNA located approximately 1 to 3 kbp upstream and DNA approximately 1 to 3 kbp downstream of a gene derived from a wild-type fungal genome, respectively, which may comprise a part or all of an endogenous secreted enzyme gene, by utilizing, for example, a genetic recombination technique and a homologous recombination technique. The gene cassette can be introduced into a genetic locus in which the wild-type fungus has a polynucleotide encoding an endogenous secreted enzyme. In this case, when the gene cassette comprises the exogenous polynucleotide encoding a GPI-addition signal sequence linked to the 3'-terminus of the polynucleotide encoding a secreted enzyme, the gene cassette can be introduced into the fungus by a homologous recombination method, so that it can be linked to the 3'-terminal side of a polynucleotide encoding a wild-type endogenous secreted enzyme, or so that it can be replaced for the polynucleotide encoding a wild-type endogenous secreted enzyme. Alternatively, the endogenous secreted enzyme of a wild-type fungus may be knocked out, and the gene cassette may be then introduced into a different gene locus from that in which the wild type fungus has a polynucleotide encoding an endogenous secreted enzyme.

The gene cassette to be introduced into a fungus may further comprise a selective marker such as a hygromycin resistance gene, an ampicillin resistance gene, a neomycin resistance gene, or a kanamycin resistance gene, in order to facilitate the selection of a modified fungus.

The gene cassette can be introduced into a fungus by utilizing a suitable vector such as an *Escherichia* coli-derived plasmid (e.g., pGEM^{(R)}-T Easy, pET22b(+), pBR322, pUC118, and pBluescript, etc.), a *Bacillus subtilis*-derived plasmid (e.g., pUB110, pTP5, etc.), and a yeast-derived plasmid (e.g., YEp13, YCp50, etc.). A gene cassette or a vector can be introduced by a method known to a person skilled in the art, such as a protoplast-PEG method, an electroporation method, and a metal treatment method.

The method for producing the modified fungus of the present invention may comprise a step of selecting the modified fungus, in addition to the modification step. The selection step can be carried out by introducing, for example, a gene cassette or a vector containing a selective marker into a fungus to transform it, as described above, and growing the transformed fungus under a selection pressure.

The method for producing the modified fungus of the present invention may comprise a step of determining whether or not a desired genetic modification has been carried out in the modified fungus, in addition to or instead of the step of selecting the modified fungus. The step can be carried out, for example, by confirming the insertion of a nucleotide according to a PCR method and electrophoresis, or by sequencing according to a PCR method, or the like.

In one aspect, the present invention relates to a method for producing a culture supernatant having a reduced amount of an endogenous secreted enzyme, comprising: a step of culturing the fungus described herein in a medium; and a step of collecting a culture supernatant having a reduced amount of the endogenous secreted enzyme from the cultured fungus.

The culture step in the method can be carried out, for example, by subjecting fungal cells to small-scale or large-scale fermentation (a continuous system, a batch system, a fed-batch system, or solid-state fermentation, etc.) in a suitable medium according to a method known to a person skilled in the art. As a medium used herein, a suitable nutrient medium supplemented with carbon and nitrogen sources and inorganic salts, for example, a commercially available medium or a medium prepared in accordance with a published prescription may be used.

The step of collecting a culture supernatant can be carried out according to a method known to a person skilled in the art, such as filtration or centrifugation. In addition, the method may optionally comprise a step of purifying or separating a specific protein from a culture supernatant, as well as the culture step and the collecting step.

The degree of the secretion amount of the endogenous secreted enzyme that is reduced in the present method is not particularly limited, but for example, the expression level may be 90% or less, 80% or less, 70% or less, 60% or less, preferably 50% or less, 40% or less, 30% or less, 20% or less, or 10% or less, compared with the expression level of a wild-type fungus. Moreover, the secretion of the endogenous secreted enzyme may be completely lost.

The culture supernatant can be used as an enzyme preparation having a reduced amount of an undesired endogenous secreted enzyme, directly or in combination with other components. Examples of such other components that may be added include water, alcohols, a preservative, an excipient, a pH adjuster, minerals, an antioxidant, a solubilizer, a binder, a suspending agent, a flavoring agent, a surfactant, and a flow promoter. These components may be used alone or in combination. Moreover, the culture supernatant may be subjected to further treatments such as filtration sterilization, concentration, and purification or separation of a desired enzyme.

The modified fungus of the present invention or the culture supernatant can be used in the production of a target substance. The endogenous secreted enzyme anchored on the surface of a cell by GPI in the modified fungus can be selected, as appropriate, depending on the target substance, and one or two or more endogenous secreted enzyme may be selected. For example, the combination of the target substance and the secreted enzyme may be a combination selected from the group consisting of the following combinations: (a) the target substance is a sugar and the secreted enzyme is glycosidase; (b) the target substance is a lipid and the secreted enzyme is lipase; (c) the target substance is a protein (or a peptide) and the secreted enzyme is protease; and (d) the target substance is a nucleic acid and the secreted enzyme is nuclease. The combination of the target substance and the secreted enzyme may preferably be a combination selected from the group consisting of the following combinations (a) to (c); more preferably, the target substance is cellobiose and the secreted enzyme is Bgll.

### 2. Method for producing target substance

In one aspect, the present invention relates to a method for producing a target substance, comprising a step of contacting a raw material substance with the modified fungus or the culture supernatant described in the above section "1. Modified fungus," to produce a target substance from the raw material substance.

In the present method, the raw material substance can be selected depending on the target substance. For example, when the target substance is a sugar, the raw material substance may be a sugar having a molecular weight higher than that of the target substance, for example, a polysaccharide such as cellulose. Likewise, when the target substance is a lipid, the raw material substance may be a lipid having a molecular weight higher than that of the target substance. When the target substance is a protein or a peptide, the raw material substance may be a protein having a molecular weight higher than that of the target substance. When the target substance is a nucleic acid, the raw material substance may be a nucleic acid having a molecular weight higher than that of the target substance.

In the present method, as described above, the combination of the target substance and the endogenous secreted enzyme is, for example, a combination selected from the group consisting of the above-described combinations (a) to (d) or combinations (a) to (c), preferably, the target substance is cellobiose and the secreted enzyme is β glucosidase.

In the production method of the present invention, the step of producing a target substance from a raw material substance is not particularly limited. For example, the target substance may be produced by culturing the modified fungus described herein in a medium containing a raw material substance, or contacting the culture supernatant described herein or an enzyme preparation containing the same with a raw material substance to be reacted with each other, under suitable conditions, for example, in water or a buffer solution, at normal temperature (10°C to 40°C, for example, 20°C to 30°C) or at low temperature (4°C to 10°C).

The production method of the present invention may comprise a step of collecting the target substance, in addition to the step of producing the target substance from the raw material substance. The collecting step can be carried out by removing the modified fungus, for example, according to centrifugation, filtration, etc. Moreover, the production method of the present invention may comprise a step of purifying or separating the raw material substance. The purification or separation step can be carried out according to an ordinary method, for example, by using any one or two or more of the following methods: chromatography such as gel filtration chromatography, ion exchange column chromatography, affinity chromatography, reverse phase column chromatography or HPLC, ammonium sulfate fractionation, ultrafiltration, and an immunoabsorption method.

### Examples

### Example 1: Construction of Bgll-GPI strain and activity measurement

### < Materials and methods >

### (Used cell strain and medium)

In the present experiment, *Trichoderma reesei* NBRC 31327 (obtained from NBRC) was used as a parent strain. AM agar medium (10 g/L glucose, 6 g/L NaNO₃, 0.52 g/L KCl, 0.52 g/L MgSO₄(7H₂O), 1.52 g/L KH₂PO₄, 2.11 g/L arginine, 5 µg/mL biotin, and Hutner's trace elements; pH 6.5) was used for cultivation to obtain conidia of *T. reesei.* Subsequently, a TM liquid medium (10 g/L glucose, 10 g/L KH₂PO₄, 6 g/L (NH₄)₂SO₄, 1 g/L MgSO₄(7H₂O), 3 g/L tri-sodium citrate(2H₂O), 5 mg/L FeSO₄(7H₂O), 2.5 mg/L MnSO₄(5H₂O), 1.4 mg/L ZnSO₄(7H₂O), and 2 mg/L CaCl₂(2H₂O); pH 6.0) was used for culturing the cells for preparation of protoplasts. In addition, a TM agar medium supplemented with 0.5 M KCl as an osmoregulating agent and 150 µg/mL Hygromycin B as a selection pressure was used as a medium for selecting a transformant. Moreover, a TM liquid medium, in which 10 g/L glucose was replaced with 10 g/L cellobiose as a carbon source, was used as a medium for measuring β-glucosidase activity.

### (Construction of strain comprising GPI-addition signal sequence linked to Bgll)

A mutant strain expressing an amino acid comprising a GPI-addition signal sequence (SEQ ID NO: 11; cDNA encoding this amino acid sequence has the nucleotide sequence as set forth in SEQ ID NO: 12) linked to the C-terminus of β glucosidase 1 (Bgll) of the NBRC 31327 strain, was prepared according to the following method.

A polynucleotide encoding a GPI-addition signal sequence was added to the bgl1 gene on the chromosome of the NBRC 31327 strain by homologous recombination. In order to produce a DNA cassette used for the homologous recombination, PCR was carried out using the genomic DNA of the NBRC 31327 as a template, and using primer sets listed in Table 1, namely, TRbgl1gpi-FC/TRbgl1gpi-R1, TRbgl1gpi-F2/TRbgl1gpi-R2, and TRbgl1gpi-F4/TRbgl1gpi-RC, to amplify the partial sequence of the bgl1 gene, the polynucleotide encoding a GPI-addition signal sequence, and the 3' UTR region of the bgl1 gene, respectively. Moreover, an hph gene encoding a hygromycin resistance marker was amplified using the plasmid pAN7.1 as a template, and the primer set TRbgl1gpi-F3/TRbgl1gpi-R3. These four PCR products were linked by fusion PCR, and were then amplified using the primer set TRbgl1gpi-F1/TRbgl1gpi-R4. The obtained product was cloned into a pGEM T-Easy vector (Promega) by TA cloning to obtain pG-bgl1-gpi. The obtained pG-bgl1-gpi was digested with the restriction enzyme NotI, and was then used to transform the NBRC 31327 strain. The NBRC 31327 strain was transformed by a protoplast-PEG method. Specifically, the NBRC 31327 strain was cultured in a TM liquid medium at 30°C at 80 rpm, and then the cells were harvested and treated with 0.4 g/L Yatalase (Takara Bio) and 0.4 g/L Cellulase Onozuka R-10 (Yakult) for protoplast formation. The transformant was selected using an AM agar medium supplemented with 0.5 M KCl and 150 µg/mL Hygromycin B. PCR was carried out using the primer set TRbgl1gpi-FC/TRbgl1gpi-R, in order to confirm that a strain comprising a polynucleotide encoding a GPI-addition signal sequence added to the bgl1 gene was obtained. An evaluation criterion in this experiment was as follows: 4.4-kb PCR product and 8.4-kb PCR product were amplified in the case of the genotype of a wild-type strain and the strain to which a polynucleotide encoding a GPI-addition signal sequence was added, respectively. The obtained strain, to which a polynucleotide encoding a GPI-addition signal sequence was added, was referred to as a Bgll-GPI strain.

The primers used in the present experiment are as follows.

### [Table 1]

**Table 1. Primers used in the present experiment**

| Primer name | Sequence (5'→73') | SEQ ID NO: |
|---|---|---|
| TRbgl1gpi-FC | ATGCGTTACCGAACAGCAGC | 1 |
| TRbgl1gpi-F1 | GCCACTGGGCCCTTTGCTAG | 2 |
| TRbgl1gpi-R1 | TTGAGGCTGGGAAGCTCGACCGCTACCGACAGAGTGCTCG | 3 |
| TRbgl1gpi-F2 | CGAGCACTCTGTCGGTAGCGGTCGAGCTTCCCAGCCTCAA | 4 |
| TRbgl1gpi-R2 | CTTCCAAGCGGAGCAGGCTCTTACCACAGCTGGGCCATGC | 5 |
| TRbgl1gpi-F3 | GCATGGCCCAGCTGTGGTAAGAGCCTGCTCCGCTTGGAAG | 6 |
| TRbgl1gpi-R3 | CTTCACCCTCCTCGCGCTAGCCAAGAGCGGATTCCTCAG | 7 |
| TRbgl1gpi-F4 | CTGAGGAATCCGCTCTTGGCTAGCGCGAGGAGGGTGAAG | 8 |
| TRbgl1gpi-R4 | TTATGGCGCGCGAGGAGATC | 9 |
| TRbgl1gpi-RC | CGCTACGCGCTCAGATTCCA | 10 |

### (Measurement of β-glucosidase activity)

The conidia (2 x 10⁷) of each of the NBRC 31327 strain and the Bgll-GPI strain were inoculated into 100 mL of a TM liquid medium containing cellobiose as a carbon source, and cultured at 30°C at 140 rpm for 2 days. A cell mass was separated from a culture supernatant by filtration, the cell mass was then freeze-dried, and the weight thereof was then measured. On the other hand, the culture supernatant was concentrated using a 10-kDa ultrafiltration filter unit (Vivacon 500, Sartorius), and the buffer was then exchanged with a 100 mM acetate buffer (pH 5.0). The resultant was reacted with 150 mM p-nitrophenyl-β-D-glucopyranoside (Sigma) (50°C, 1 hour), and the absorbance (Abs = 400 nm) was then measured.

### < Results >

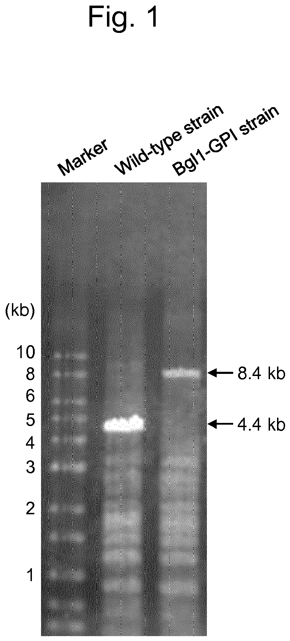

As described above, the cells were transformed with the partial sequence of the bgl1 gene, the polynucleotide encoding a GPI-addition signal sequence, the 3' UTR region of the bgl1 gene, and an pG-bgl1-gpi comprising hph gene, and selected using hygromycin, so as to construct a mutant strain of a *Trichoderma reesei* NBRC 31327 strain. PCR was performed on the wild-type strain and the mutant strain, and shift-up caused by sequence insertion of the marker gene and the polynucleotide encoding a GPI-addition signal sequence was observed, so as to confirm that a strain in which the polynucleotide encoding a GPI-addition signal sequence was added to the bgl1 gene was obtained (Figure 1).

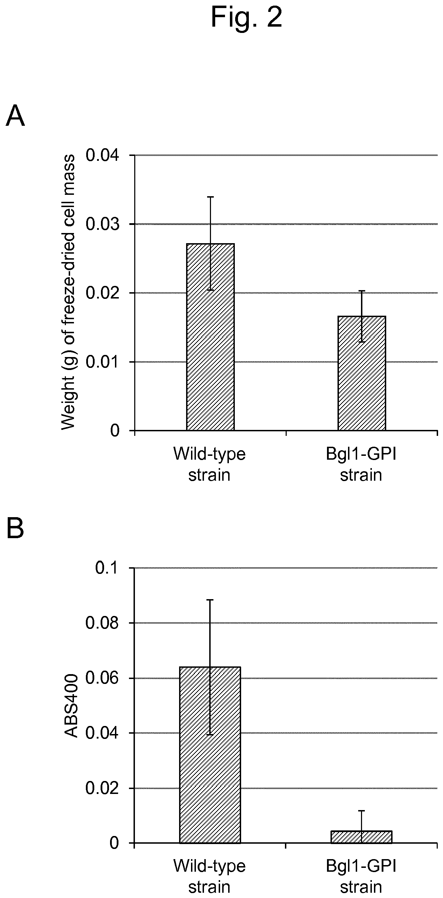

Subsequently, the NBRC 31327 strain (wild-type strain) and the obtained mutant strain (Bgll-GPI strain) were cultured in a TM liquid medium containing cellobiose as a carbon source for 2 days. After cultivation, a cell mass was separated from a culture supernatant by filtration, the cell mass was then freeze-dried, and the weight thereof was measured. As a result, it was found that the weight of the freeze-dried cell mass of the Bgll-GPI strain tended to be lower than that of the wild-type strain, suggesting that the viability of the Bgll-GPI strain is slightly lower than that of the wild-type strain (Figure 2A).

Subsequently, the Bgl activity of the culture supernatant of each of the wild-type strain and the Bgll-GPI strain was measured using p-nitrophenyl-β-D-glucopyranoside as a substrate. As a result, the Bgl activity of the culture supernatant of the Bgll-GPI strain was significantly lower than that of the wild-type strain (Figure 2B).

### Example 2: Comparison of various enzyme activities in Bgll-GPI strain

### < Materials and methods >

The spore (1 x 10⁷) of each of the Bgll-GPI strain obtained in Example 1 and the NBRC31327 strain as a wild-type strain was inoculated into 150 mL of a medium, and was cultured at 30°C at 163 rpm for 2 days. A medium having the following composition, which was adjusted to pH 6.0 with 10 N NaOH, was used.

### [Table 2]

**Table 2. Medium composition**

| | |
|---|---|
| Cellobiose | 10 g/L |
| KH₂PO₄ | 10 g/L |
| (NH₄)₂SO₄ | 6 g/L |
| MgSO₄▪7H₂O | 1 g/L |
| Trisodium citrate·2H₂O | 3 g/L |
| Trace element solution | 1 ml/L |

After cultivation, a cell mass was separated from a culture supernatant using a glass filter (IWAKI, 3G1), the cell mass was then freeze-dried, and the weight thereof was then measured. In addition, the enzyme activities (CMCase activity, CBH activity, and BGL activity) of the culture supernatant were measured as follows, and the enzyme activity per protein mass was calculated. Specifically, the BGL activity was measured by quantifying glucose generated as a result of a reaction with a substrate cellobiose concentration of 1% at pH 5.0 at 50°C for 10 minutes, by an enzyme method (Wako Pure Chemical Industries, Ltd.: Glucose CII Test Wako). The CMC degrading activity was measured by quantifying reducing sugar generated by a reaction performed using the carboxymethyl cellulose of Sigma at a substrate concentration of 1%, pH 5.0, and 50°C for 10 minutes, according to a quantification method (DNS method) using 3,5-dinitrosalicylic acid, using glucose as a reference. The CBH activity was measured by quantifying reducing sugar generated by a reaction performed using the KC Flock (W-400G) manufactured by NIPPON PAPER INDUSTRIES CO., LTD. or Sigma Avicel (PH-101), at a substrate concentration of 1(w/v) %, pH 5.0, 50°C for 10 minutes, according to a quantification method (DNS method) using 3,5-dinitrosalicylic acid, using glucose as a reference.

### < Results >

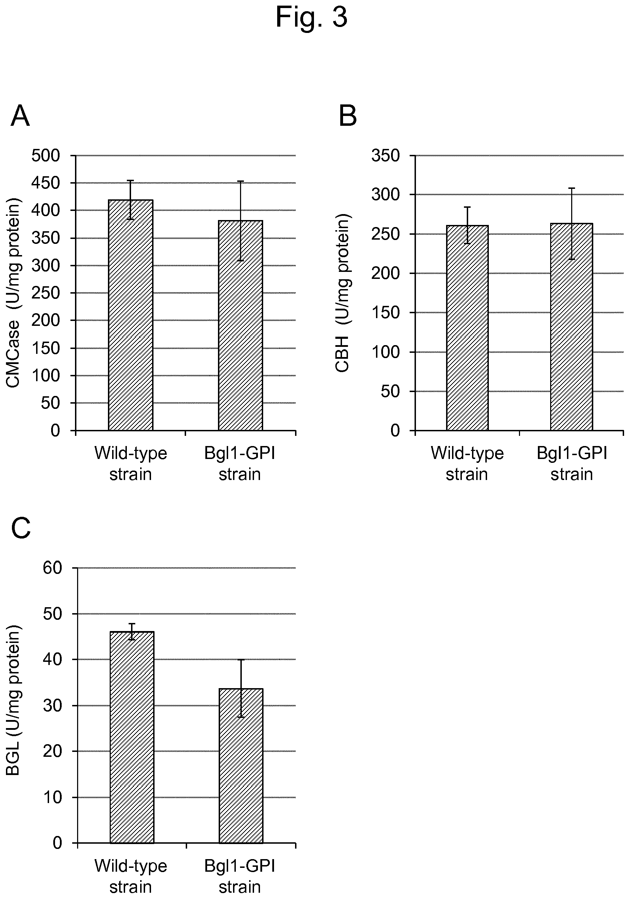

The weight of a freeze-dried cell mass of the wild-type strain and the Bgll-GPI strain was 0.12 g and 0.1 g, respectively. The results of measuring the enzyme activities of the culture supernatant are shown in Figure 3. As shown in Figure 3, the CMCase activity (A) and the CBH activity (B) was not decreased, whereas only the BGL activity (C) was significantly decreased. Accordingly, it was demonstrated that the release of Bgl1 into the culture supernatant was suppressed by addition of the GPI anchor to Bgll. Example 3: Comparison of cellobiose productivity in Bgll-GPI strain and wild-type strain

### < Materials and methods >

By the same method as described in Example 2, the cell strains were each cultured, and a cell mass was separated from a culture supernatant using a glass filer. Subsequently, the culture supernatant was reacted with cotton cellulose obtained by crushing absorbent cotton manufactured by Kakui (wherein the reaction was carried out using 0.25% cotton in an acetate buffer [pH 5.0] at 50°C), and the reaction mixture was sampled over time (0 hour, 4 hours, and 10 hours). The obtained reaction solution of the culture supernatant and the cotton cellulose was filtrated with a cellulose acetate membrane filter with an aperture of 0.2 µm manufactured by Toyo Roshi Kaisha, Ltd. (ADVANTEC, 25CS020AS). The filtrate was measured by HPLC, and concentration of cellobiose and glucose and the ratio between them were calculated from the peak areas of glucose and cellobiose.

### < Results >

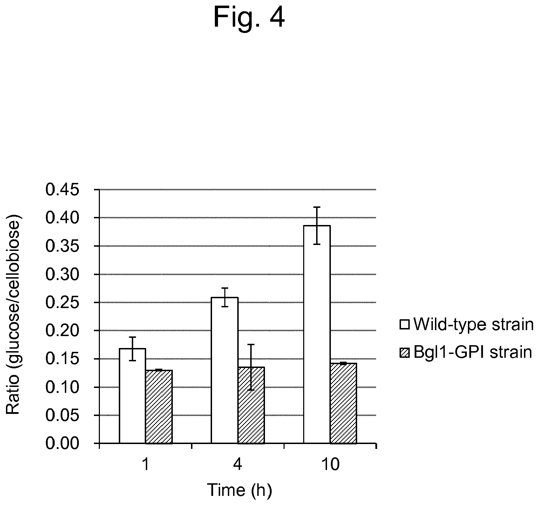

The results of measuring the production ratio of glucose/cellobiose are shown in Figure 4. The ratio of glucose to cellobiose produced from the cotton cellulose was increased in the wild-type strain over time, whereas it was not increased in the Bgll-GPI anchor added strain. These results suggests that the enzyme produced by the Blg1-GPI anchored strain is effective for the production of cellobiose from cellulose and suppression of the production of glucose.

### Industrial Applicability

According to the present invention, a culture supernatant having a reduced amount of a specific endogenous secreted enzyme, may be obtained, and the obtained culture supernatant may be directly used as an enzyme preparation. Moreover, a combination of the secreted enzymes can be easily customized by applying the present method to various enzymes secreted by fungi, and thus, enzyme preparations suitable for various purposes can be produced. The present invention has great industrial applicability, since the efficiency of substance production may be improved by using an enzyme preparation having a reduced amount of an undesired enzyme.

All publications, patents and patent applications cited in the present description are incorporated herein by reference in their entirety.

## Claims

1. A modified fungus, expressing a fusion polynucleotide comprising an exogenous polynucleotide encoding a glycosylphosphatidylinositol (GPI)-addition signal sequence linked to the 3'-terminal side of a polynucleotide encoding an endogenous secreted enzyme, and thereby, the secreted enzyme is anchored on the cell surface by GPI.

2. The fungus according to claim 1, which belongs to filamentous fungi.

3. The fungus according to claim 2, which belongs to filamentous fungi selected from the group consisting of the genus Trichoderma, the genus Aspergillus, the genus Penicillium, the genus Monascus, and the genus Thermoascus.

4. The fungus according to claim 3, which belongs to the species *Trichoderma reesei.*

5. The fungus according to any one of claims 1 to 4, wherein the secreted enzyme is selected from the group consisting of glycosidase, lipase, protease, and nuclease.

6. The fungus according to any one of claims 1 to 5, which is used in the production of a target substance, wherein the target substance and the secreted enzyme are selected from the group consisting of the following combinations:
(a) the target substance is a sugar and the secreted enzyme is glycosidase;
(b) the target substance is a lipid and the secreted enzyme is lipase;
(c) the target substance is a protein and the secreted enzyme is protease; and
(d) the target substance is a nucleic acid and the secreted enzyme is nuclease.

7. The fungus according to claim 6, wherein the target substance is cellobiose and the secreted enzyme is β glucosidase.

8. A method for producing a fungal culture supernatant having a reduced amount of an endogenous secreted enzyme, comprising:
a step of culturing the fungus according to any one of claims 1 to 5 in a medium; and
a step of collecting a culture supernatant having a reduced amount of an endogenous secreted enzyme from the cultured fungus.

9. A method for producing a target substance, comprising
a step of contacting a raw material substance with the fungus according to any one of claims 1 to 5 or the fungal culture supernatant according to claim 8, to produce a target substance from the raw material substance.

10. The method according to claim 9,
wherein the target substance and a secreted enzyme are any of the following combinations:
(a) the target substance is a sugar and the secreted enzyme is glycosidase;
(b) the target substance is a lipid and the secreted enzyme is lipase;
(c) the target substance is a protein and the secreted enzyme is protease; and
(d) the target substance is a nucleic acid and the secreted enzyme is nuclease.

11. The method according to claim 10, wherein the target substance is cellobiose and the secreted enzyme is β glucosidase.

12. The method according to any one of claims 9 to 11, comprising a step of collecting the target substance.

13. A method for producing a modified fungus, in which an endogenous secreted enzyme is anchored on the surface of a cell by glycosylphosphatidylinositol (GPI),
wherein the method comprises a step of modifying a fungus to express a fusion polynucleotide comprising an exogenous polynucleotide encoding a GPI-addition signal sequence linked to the 3'-terminal side of a polynucleotide encoding the endogenous secreted enzyme.

14. The method according to claim 13, wherein the fungus belongs to the species *Trichoderma reesei.*

15. The method according to claim 13 or 14, wherein the secreted enzyme is selected from the group consisting of glycosidase, lipase, protease, and nuclease.
